# EUROPEAN PATENT APPLICATION

(11) **EP 1 632 251 A1**
(43) Date of publication of application: **08.03.2006**
(21) Application number: 04425663.4
(22) Date of filing: 02.09.2004
(51) Int. Cl.: A61L 9/012, A61L 9/04

(54) **Product for deodorizing, perfuming or sanitizing of enclosed spaces comprising at least two carriers for volatile substances**

(71) Applicant: Deoflor S.p.A., 27029 Vigevano (Pavia) (IT)
(72) Inventor: Ruffina, Laura, 27036 Mortara (IT)
(74) Representative: Modiano, Micaela Nadia

(57) **Abstract**

A deodorizing or fragrancing or sanitizing product or the like (1a, 1b) for enclosed spaces, which comprises at least two solid or semisolid carriers (2a, 3a; 2b, 3b), which contain a volatile active substance. At least one of the two carriers (2a, 3a; 2b, 3b) is transparent or semitransparent or translucent, and the two carriers are in contact by at least one of their faces.

## Description

The present invention relates to a deodorizing or fragrancing or sanitizing product or the like for enclosed spaces.

In the field of deodorizing or fragrancing or sanitizing products for enclosed spaces, there is continuing research aimed at achieving an increasingly satisfactory aesthetic presentation by working both on the carrier that contains the volatile active substance to be diffused into the enclosed space and on the container in which the carrier is inserted.

For this purpose, transparent and colored containers have been proposed which contain a carrier, generally in solid or gel form, that is colored differently from the container, so as to obtain different aesthetic effects based on the combination of the color of the container and the color of the carrier that is visible through the container.

In other cases, the container has grooves into which the appropriately colored carrier is poured.

In other cases, a single-color carrier is inserted in a translucent or transparent multicolored container.

Containers are also known which have a containment chamber that is physically divided into a plurality of compartments, into which the carrier is arranged, said carrier being variously colored according to the compartment.

Liquid products are also known which utilize the principle of phase separation by emulsion in order to offer a two-color product.

The aim of the present invention is to provide a deodorizing or fragrancing or sanitizing product or the like for enclosed spaces with a solid or semisolid carrier, in which the two-color or multicolored effect, or more generally an effect arising from the combination of optically diversified parts, is obtained directly by means of the carrier, without necessarily requiring the cooperation of the container in order to separate the various optically different components.

Within this aim, another object of the invention is to provide a product that has a two-color or multicolored or optically diversified effect that is substantially stable over time.

Another object of the invention is to provide a product in which the two-color or multicolored or optically diversified effect obtained directly by means of the carrier may in any case be combined with the color of the container, so as to further increase the various possibilities of presentation and differentiation of these kinds of products.

Another object of the invention is to provide a product in which the two-color or multicolored or optically diversified effect can be achieved in a relatively simple manner without complicating excessively the production cycle of the product.

This aim and these and other objects that will become better apparent hereinafter are achieved by a deodorizing or fragrancing or sanitizing product or the like for enclosed spaces, characterized in that it comprises at least two solid or semisolid carriers, which contain a volatile active substance, at least one of said two carriers being transparent or semitransparent or translucent, said two carriers being in contact by at least one of their faces.

Further characteristics and advantages of the invention will become better apparent from the description of two preferred but not exclusive embodiments of the product according to the invention, illustrated by way of non-limiting example in the accompanying drawings, wherein:
Figure 1 is a front elevation view of the product according to the invention in a first embodiment, inserted within a transparent or translucent container;
Figure 2 is a sectional view of Figure 1, taken along the line II-II;
Figure 3 is a front elevation view of the product according to the invention in a second embodiment.

With reference to the figures, the product according to the invention, generally designated by the reference numerals 1a and 1b in the two embodiments, comprises at least two carriers 2a, 3a and 2b, 3b, which are solid or semisolid (for example gels) and contain, in a per se known manner, a fragrancing, deodorizing or sanitizing volatile active substance to be dispersed in the enclosed space. At least one of the two carriers 2a, 3a, 2b, 3b is transparent or semitransparent or translucent and is preferably colored differently from the other carrier or carriers. The at least two carriers 2a, 3a, 2b, 3b are in contact by at least one of their faces, so as to constitute, as a whole, a two-color carrier, in the case of two carriers, or a multicolored carrier, in the case of multiple carriers.

It should be noted that the at least two carriers may also be of the same color. In this case, the optical distinction between the two or more carriers can be obtained by means of a different degree of transparency of the carriers that compose the product.

More particularly, the carriers may be all transparent and/or variously colored or may be partly transparent and partly translucent or opaque.

The at least two carriers are substantially chemically stable with respect to each other, so as to avoid as much as possible migration of color from one carrier to the other, in order to keep the optical distinction among the carriers unchanged over time.

This is preferably achieved by providing the carriers 2a, 3a, 2b, 3b by means of a material that is selected among polyamides, polyesters, terminal starches, aliphatic hydrocarbons with a chain comprising 6 to 18 carbon atoms, polyurethanes, elastomers, and derivatives and mixtures thereof.

As an alternative, two carriers that are in mutual contact may be provided so that one is water-based, by using for example carrageenan, gellan gum, xanthan gum or the like, and mixtures thereof, and the other carrier has an anhydrous base, by using for example polyamides, waxes or the like and mixtures thereof.

The term "waxes" is used here to designate materials that have an ointment-like appearance and are characterized by a semisolid consistency at 25°C. Typical examples of waxes are selected from the group that comprises vaselines, silicone oils, natural waxes (animal and vegetable waxes), mono-, di- and triglycerides of saturated or unsaturated fatty acids generally with more than ten carbon atoms in a chain, polyethylene glycols with a molecular weight of generally more than 400, natural and synthetic fatty acids, sorbitan esters of fatty acids, polyoxyethylated sorbitan esters of fatty acids, carboxypolymethylene, and mixtures thereof. A preferred wax is constituted by stearates of alkaline and alkaline-earth metals, even more preferably sodium stearate.

Optionally, the exposed surface and/or the surface of the carriers that are in mutual contact may be coated with a transparent and evaporable polymeric film-forming agent, which further increases the aesthetic properties of the product and at the same time performs an impermeabilizing function, which hinders the migration of the color from one carrier to the other, allowing to use even substances that are not completely stable with respect to each other to produce the carriers.

Said film-forming agent is, upon application, in liquid or semisolid form and is preferably a polymeric resin, preferably a polyamide resin, that contains residues of fatty acids, preferably bifunctional fatty acids, and advantageously also an alcohol component that facilitates its application and is present in an amount comprised between 15 and 40% by weight on the total weight of the mixture.

The film-forming agent advantageously has a flash point of more than 205 °C and a viscosity equal to 2000 cps at 160 °C.

The aesthetic effect obtained by means of the optical diversification of the at least two carriers that are used may be increased by introducing, in one or more of the carriers that compose the product, additives and/or particles that can be optically distinguished from the remaining part of the carrier in which they are incorporated, such as for example pearlescent agents, glitters, florescent dyes, markers or others.

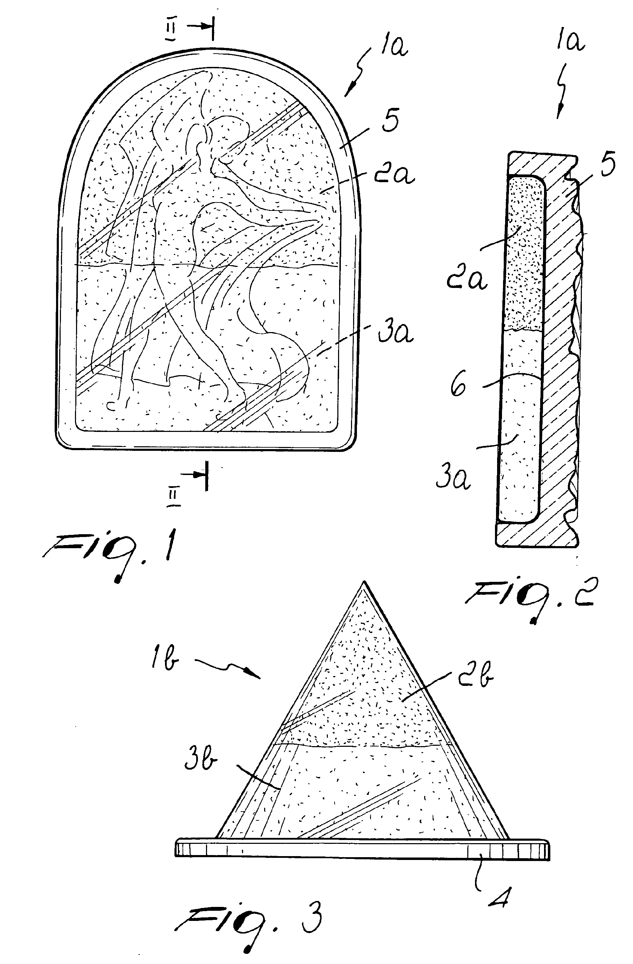

The carriers may have a formulation that makes them self-supporting, so that they do not need to be arranged inside a container. In this case, the liquid or semiliquid carriers can be poured into appropriate molds in order to obtain the intended shape and be extracted from said molds after they have stabilized. The product may have various configurations, such as for example shapes that reproduce animals, flowers or other subjects, or may have geometric shapes, as shown for example in Figure 3, in which the product 1b is shaped like a cone that is arranged on a base disc 4.

The carriers may also be arranged inside a container 5, as shown in Figures 1 and 2. Said container 5 has a compartment 6 occupied by the carriers 2a, 3a and is preferably at least partially transparent or translucent so as to allow to view the carriers 2a, 3a through it. In this case, the container can be colored and/or shaped and may bear images, optionally in relief, so as to further enhance the aesthetic appearance of the carriers 2a, 3a that it contains.

Optionally, the above cited optically distinguishable particles may also be arranged between the container 5 and/or between the carriers 2a, 3a.

The carriers are preferably poured, in the liquid or semiliquid state, into the compartment 6 and are allowed to stabilize.

More particularly, the various carriers are arranged inside the compartment 6 by pouring in succession the various carriers inside the compartment 6 and by waiting for the previously poured carrier to stabilize before pouring a carrier.

Depending on the aesthetic requirements, the various carriers may be arranged horizontally side by side or be vertically superimposed.

The product according to the invention may be completed by means of supporting elements of various kinds according to the intended use. For example, it can be provided with hooks or double-adhesive elements in order to be hung in the enclosed space to be deodorized or fragranced or sanitized.

In practice it has been found that the deodorizing or fragrancing or sanitizing product fully achieves the intended aim, since it allows to achieve a two-color or multicolored effect or more generally an effect that arises from the combination of optically diversified parts without necessarily requiring, in order to separate the optically different components, the cooperation of the container in which the carriers are inserted.

The container, if present, can be used to enhance the aesthetic effect obtained directly by means of the carriers of the product according to the invention.

The product thus conceived is susceptible of numerous modifications and variations, all of which are within the scope of the inventive concept; all the details may further be replaced with other technically equivalent elements.

In practice, the dimensions and the contingent shapes may be any according to requirements.

Merely by way of indication, some examples of products according to the invention are given hereafter.

### Example 1

| Ingredients of first step | % (by weight) |
|---|---|
| Citric 055200733 fragrance | 20 |
| Versagel F-1100 | 80 |
| Yellow dye | traces |

Versagel F-1100 is supplied by Penreco

Fragrance 055200734 is supplied by Sensient Fragrances

Preparation: the Versagel F-1100 product, which constitutes the carrier, is heated until it liquefies completely (90 °C). The temperature is lowered to 75-80 °C and then the Fragrance 055200733, which constitutes the volatile active substance, and the dye, are introduced under agitation. After an appropriate mixing time, the preparation is poured into an appropriate mold until the product cools completely and consequently solidifies.

| Ingredients of step 2 | % |
|---|---|
| Marine 553 fragrance | 40 |
| Sylvaclear A200 | 60 |
| Blue dye | traces |

Sylvaclear A200 is supplied by Arizona Chemical

Marine 553 fragrance is supplied by IFF

Preparation: the Sylvaclear A200 product, which constitutes the carrier, is heated until it liquefies completely (60 °C), then the Marine 553 fragrance, which constitutes the volatile active substance, and the dye, are introduced under agitation. After an appropriate mixing time and after further lowering the temperature to 50 °C, the preparation is poured into the same mold and in contact with the product of step 1, until the product cools completely and consequently solidifies. The part is then extracted from the mold and has a two-color appearance and two different fragrances that are not in contrast with each other; the part is then arranged on an appropriate support.

### Example 2

| Ingredient | % |
|---|---|
| AD 9115 fragrance | 40 |
| Sylvaclear A200 | 60 |
| Dye | traces |

Sylvacote 228E as a film-forming coating agent

Sylvaclear A200 is supplied by Arizona Chemical

Fragrance AD 9115 is supplied by Quest

Sylvacote 228E is supplied by Arizona Chemical

Preparation: the Sylvaclear A200 product, which constitutes the carrier, is heated until it liquefies completely (60 °C); then the AD 9115 fragrance, which constitutes the volatile active substance, is introduced under agitation. The product is then divided into two different fractions and dyed with two different colors (for example, orange and yellow). After an appropriate mixing time, one fraction of the preparation is poured into an appropriate container until the product cools completely and consequently solidifies. The end part of the product is coated (by means of a brush) with the solution of Sylvacote 2228E, which constitutes the coating/barrier substance. The product is allowed to dry until the barrier agent hardens completely. The second fraction of the preparation, which is differently colored, is poured into the same container and in contact with the region that has been rendered impermeable, until the product cools completely and consequently solidifies.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A deodorizing or fragrancing or sanitizing product or the like for enclosed spaces, **characterized in that** it comprises at least two solid or semisolid carriers, which contain a volatile active substance, at least one of said two carriers being transparent or semitransparent or translucent, said at least two carriers being in mutual contact by at least one of their faces.

2. The product according to claim 1, **characterized in that** said at least two carriers have mutually different colors.

3. The product according to claim 1, **characterized in that** said at least two carriers are chemically stable with respect to each other.

4. The product according to one or more of the preceding claims, **characterized in that** said at least two carriers are made of a material selected among polyamides, polyesters, terminal starches, aliphatic hydrocarbons with a chain comprising 6 to 18 carbon atoms, polyurethanes, elastomers, and derivatives and mixtures thereof.

5. The product according to one or more of the preceding claims, **characterized in that** said at least two carriers are respectively water-based and anhydrous-based.

6. The product according to one or more of the preceding claims, **characterized in that** at least one of said carriers incorporates additives and/or particles that can be distinguished optically from the remaining part of the carrier in which they are incorporated.

7. The product according to one or more of the preceding claims, **characterized in that** a film-forming agent is interposed between said at least two carriers.

8. The product according to one or more of the preceding claims, **characterized in that** at least part of the exposed surface of said carriers is coated with a film-forming agent.

9. The product according to one or more of the preceding claims, **characterized in that** said supports are self-supporting.

10. The product according to one or more of the preceding claims, **characterized in that** said supports are inserted in a container that is at least partially transparent or translucent.

11. The product according to one or more of the preceding claims, **characterized in that** bodies and/or additives and/or particles that are optically distinguishable from said carriers and/or from said container are arranged between said at least two carriers and/or between said carriers and said container.

12. The product according to one or more of the preceding claims, **characterized in that** it is obtained by successive pourings of the various carriers, in the liquid or semiliquid state, into a mold, allowing the previously poured carrier to stabilize in each instance before pouring the next carrier.

13. The product according to one or more of the preceding claims, **characterized in that** said supports are superimposed vertically.

14. The product according to one or more of the preceding claims, **characterized in that** said supports are arranged side by side horizontally.

## Amended claims

### Amended claims in accordance with Rule 86(2) EPC.

**1.** A deodorizing or fragrancing or sanitizing product (1a;1b) for enclosed spaces, comprising at least two solid or semisolid carriers (2a,3a;2b,3b), which contain a volatile active substance, at least one of said two carriers being transparent or semitransparent or translucent, said at least two carriers (2a,3a;2b,3b) being in mutual contact by at least one of their faces, said at least two carriers (2a,3a;2b,3b) being respectively water-based and anhydrous-based.

**2.** The product according to claim 1, **characterized in that** said at least two carriers (2a,3a;2b,3b) have mutually different colors.

**3.** The product according to claim 1, **characterized in that** said at least two carriers (2a,3a;2b,3b) are chemically stable with respect to each other.

**4.** The product according to one or more of the preceding claims, **characterized in that** said at least two carriers (2a,3a;2b,3b) are made of a material selected among polyamides, polyesters, terminal starches, aliphatic hydrocarbons with a chain comprising 6 to 18 carbon atoms, polyurethanes, elastomers, and derivatives and mixtures thereof.

**5.** The product according to one or more of the preceding claims, **characterized in that** at least one of said carriers (2a,3a;2b,3b) incorporates additives and/or particles that can be distinguished optically from the remaining part of the carrier in which they are incorporated.

**6.** The product according to one or more of the preceding claims, **characterized in that** a film-forming agent is interposed between said at least two carriers (2a,3a;2b,3b).

**7.** The product according to one or more of the preceding claims, **characterized in that** at least part of the exposed surface of said carriers (2a,3a;2b,3b)is coated with a film-forming agent.

**8.** The product according to one or more of the preceding claims, **characterized in that** said carriers (2a,3a;2b,3b) are self-supporting.

**9.** The product according to one or more of the preceding claims, **characterized in that** said carriers (2a,3a;2b,3b) are inserted in a container (5) that is at least partially transparent or translucent.

**10.** The product according to one or more of the preceding claims, **characterized in that** bodies and/or additives and/or particles that are optically distinguishable from said carriers and/or from said container (5) are arranged between said at least two carriers (2a,3a;2b,3b) and/or between said carriers and said container.
